# FASCICULE DE BREVET EUROPEEN

(11) **EP 4 101 424 B1**
(45) Date de publication et mention de la délivrance du brevet: **25.03.2026**
(21) Numéro de dépôt: 22177578.6
(22) Date de dépôt: 07.06.2022
(51) Int. Cl.: A61F 5/14

(54) **DISPOSITIF SUPPORT, ÉQUIPEMENT ET PROCÉDÉ POUR RÉPARER OU MODIFIER UN DISPOSITIF MÉDICAL DE TYPE ORTHÈSE OU PROTHÈSE**
HALTEVORRICHTUNG, AUSRÜSTUNG UND VERFAHREN ZUM REPARIEREN ODER ABÄNDERN EINER MEDIZINISCHEN VORRICHTUNG VOM TYP ORTHESE ODER PROTHESE
SUPPORT DEVICE, SYSTEM AND METHOD FOR REPAIRING OR MODIFYING A MEDICAL DEVICE SUCH AS AN ORTHOSIS OR PROSTHESIS

(30) Priorité: 08.06.2021 FR 2106050
(43) Date de publication de la demande: 14.12.2022
(73) Titulaire: TROD-3OD, 44250 Saint-Brevin-Les-Pins (FR)
(72) Inventeur: FAVREAU, Gwenaël, 44250 SAINT BREVIN LES PINS (FR)
(74) Mandataire: Jacobacci Coralis Harle

(56) Documents cités:
- US-A1- 2016 288 440
- US-B2- 8 170 705
- SHAHAR FARAH SYAZWANI ET AL: "A review on the orthotics and prosthetics and the potential of kenaf composites as alternative materials for ankle-foot orthosis", JOURNAL OF THE MECHANICAL BEHAVIOR OF BIOMEDICAL MATERIALS, ELSEVIER, AMSTERDAM, NL, vol. 99, 24 July 2019 (2019-07-24), pages 169 - 185, XP085775118, ISSN: 1751-6161, [retrieved on 20190724], DOI: 10.1016/J.JMBBM.2019.07.020

## Description

### Domaine technique de l'invention

La présente invention concerne le domaine technique des dispositifs médicaux de type orthèse ou prothèse.

Elle concerne plus particulièrement les dispositifs support, les équipements et les procédés pour réparer ou modifier un dispositif médical de type orthèse ou prothèse.

### Etat de la technique

D'une manière générale, les dispositifs médicaux de type orthèses sont des appareillages adaptés pour aider une fonction, une articulation ou un membre.

Plus particulièrement, les dispositifs médicaux de type orthèses plantaires (semelles ou orthoplasties par exemple) ont pour fonction de corriger une lacune biomécanique, posturale ou proprioceptive.

De telles orthèses plantaires comprennent - une première surface de dispositif, anatomique, destinée à venir recouvrir une partie de corps d'un individu (le dessous du pied pour une semelle, et le pourtour d'un doigt de pied pour une orthoplastie), et - une seconde surface de dispositif, opposée à ladite première surface de dispositif.

Ces dispositifs médicaux peuvent être réalisés en différents matériaux, par exemple en matériaux élastomères thermoplastiques (TPE, TPU...) sensibles à la température. Et ils peuvent être fabriqués par des technologies thermoplastiques de type extrusion, par exemple en impression 3D.

A titre d'exemple, Le document US 8 170 705 décrit une installation pour fabriquer une semelle orthopédique en plusieurs couches superposées.

Cette installation comprend un plateau support et des moyens de pressage qui comprennent un bloc chauffant.

Mais de tels dispositifs médicaux de l'état de la technique sont difficilement réparables ou modifiables, et, lorsqu'ils ne conviennent plus, ils sont généralement remplacés par un nouvel équipement.

Cela entraine des problèmes de coût et génère des problèmes de traitement des déchets, les matériaux constitutifs étant difficilement recyclablesPour des raisons tant économiques qu'écologiques, et pour répondre aux demandes éventuelles d'adaptation de l'orthèse ou de la prothèse, il existe donc un besoin de disposer d'un matériel ou d'un équipement permettant de réparer ou de modifier ce genre de dispositif médical.

Et le même problème peut se retrouver pour d'autres dispositifs médicaux réalisés à base des mêmes matériaux et techniques de fabrication, tels que des orthèses de correction optique ou des prothèses pour le remplacement d'un membre ou d'une articulation.

### Présentation de l'invention

Afin de remédier aux inconvénients précités de l'état de la technique, la présente invention propose un dispositif support pour la réception d'un dispositif médical de type orthèse ou prothèse tel que défini par la revendication 1

Par température de ramollissement on entend une température à laquelle le matériau subit un changement d'état, d'un état solide vers un état semi-liquide ou pâteux.

D'autres caractéristiques non limitatives et avantageuses du dispositif support conforme à l'invention, prises individuellement ou selon toutes les combinaisons techniquement possibles, sont montrées dans les revendications 2-7.

La présente invention concerne encore un équipement pour réparer ou modifier un dispositif médical de type orthèse ou prothèse, selon les revendications 8-12.

La présente invention concerne encore un procédé, tel que défini par la revendication 13, pour réparer ou modifier un dispositif médical de type orthèse ou prothèse, au moyen d'un équipement tel que défini ci-dessus.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
- la figure 1 est une vue générale d'une forme de réalisation possible d'un équipement conforme à l'invention pour réparer ou modifier un dispositif médical de type semelle ou orthoplastie, cet équipement comprenant notamment un dispositif support pour la réception du dispositif médical, un dispositif de distribution d'un matériau de travail et un dispositif de façonnage de ce matériau de travail ;
- la figure 2 est une vue isolée du dispositif support de dispositif médical faisant partie de l'équipement illustré sur la figure 1 ;
- la figure 3 est une vue de côté d'une autre forme de réalisation possible d'un dispositif support de dispositif médical conforme à l'invention ;
- la figure 4 est une vue isolée du dispositif de distribution faisant partie de l'équipement illustré sur la figure 1 ;
- la figure 5 est une vue éclatée du dispositif de distribution illustré sur la figure 4 ;
- la figure 6 est une vue schématique d'une partie du dispositif de distribution illustré sur les figures 4 et 5 montrant la vis de transfert du matériau de travail ;
- la figure 7 est une vue isolée du dispositif de façonnage faisant partie de l'équipement illustré sur la figure 1 ;
- la figure 8 est une vue éclatée du dispositif de façonnage illustré sur la figure 7.

Il est à noter que, sur ces figures, les éléments structurels et/ou fonctionnels communs aux différentes variantes peuvent présenter les mêmes références.

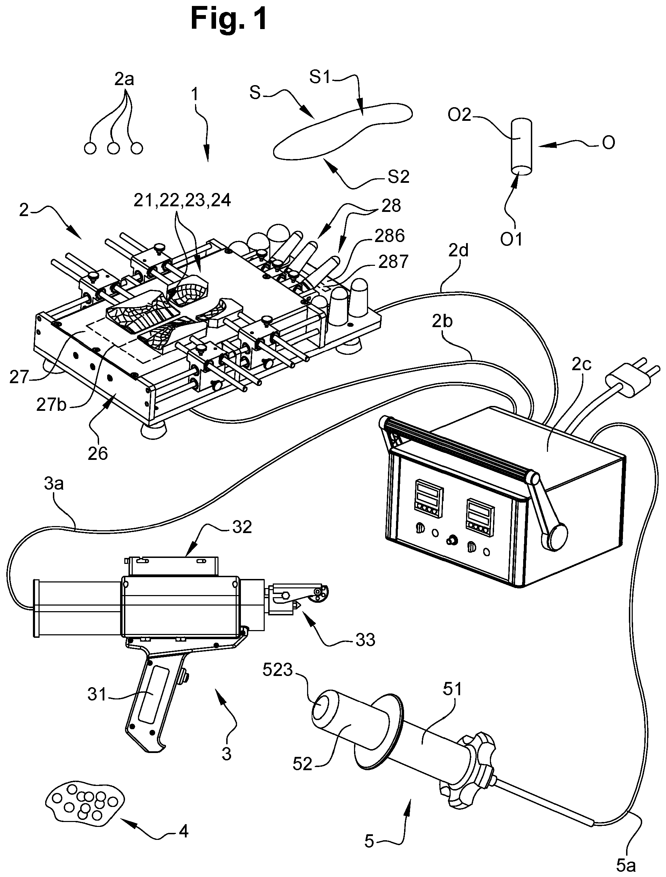

L'équipement 1 illustré sur la figure 1 est adapté pour réparer ou modifier un dispositif médical de type semelle S ou orthoplastie O qui est réalisé en matériau thermoformé comprenant une température de ramollissement, et lequel dispositif médical de type semelle S ou orthoplastie O comprend - une première surface de dispositif S1, O1, anatomique, destinée à venir recouvrir une partie de corps d'un individu, et - une seconde surface de dispositif S2, O2, opposée à ladite première surface de dispositif.

Un dispositif médical de type semelle S et un dispositif médical de type orthoplastie O sont représentés schématiquement sur la figure 1.

Le dispositif médical de type semelle S ou orthoplastie O peut par exemple être réalisé en un matériau plastique sensible à la chaleur, tel qu'un matériau élastomère thermoplastique (TPE), genre Polyuréthane thermoplastique (TPU) par exemple. Pour certaines applications, on peut aussi envisager l'utilisation d'acide polylactique (PLA).

La température de ramollissement de tels matériaux, qui correspond à la température de changement d'état du solide vers le pâteux (ou semi-liquide) peut être de l'ordre de 60 à 250°C (plus généralement de l'ordre de 180 à 250°C).

Pour une semelle S, la première surface de dispositif S1 correspond à la surface de dessus de la semelle contre laquelle vient reposer le dessous du pied d'un individu ; et la seconde surface de dispositif S2 correspond à la surface de dessous de la semelle.

Pour une orthoplastie O, de forme générale tubulaire, la première surface de dispositif O1 correspond à la surface interne destinée à venir en contact avec la surface externe d'un doigt de pied ; et la seconde surface de dispositif O2 correspond à la surface externe.

L'épaisseur du dispositif médical de type semelle S ou orthoplastie O, entre la première surface de dispositif S1, O1 et la seconde surface de dispositif S2, O2, peut être régulière ou non ; cette épaisseur peut être de quelques millimètres, jusqu'à 1 ou 2 cm.

L'équipement 1 conforme à l'invention illustré sur la figure 1 comprend un dispositif support 2 pour la réception du dispositif médical de type semelle S ou orthoplastie O, un dispositif de distribution 3 d'un matériau de travail 4, et un dispositif de façonnage 5 de ce matériau de travail.

Le matériau de travail 4 est un matériau identique, similaire ou tout au moins compatible avec le matériau constitutif du dispositif médical de type semelle S ou orthoplastie O. Il est adapté pour réparer et/ou modifier ce dispositif médical de type semelle S ou orthoplastie O, et il se présente sous la forme d'une poudre, de paillettes, de billes, ou d'un bloc de matière. A titre d'exemple on utilise un matériau du genre élastomère thermoplastique (TPE), sous forme de billes d'un diamètre de l'ordre de 1 à 3 mm.

Par les notions de réparation/modification du dispositif médical de type semelle S ou orthoplastie O, on entend une modification de l'appareillage initial par enlèvement(s) ou rajout(s) de matière identique ou différente.

La fonction de cet équipement 1 est :
- d'assurer un support optimal pour intervenir sur une zone du dispositif médical de type semelle S ou orthoplastie O qui doit être modifiée ou réparée,
- de permettre de porter le matériau de travail 4, ainsi que la zone d'intervention sur le dispositif médical de type semelle S ou orthoplastie O, à une température au moins égale à leur température de ramollissement,
- de permettre la dépose du matériau de travail 4 ramolli sur la zone d'intervention du dispositif médical de type semelle S ou orthoplastie O, et
- de façonner (c'est-à-dire étaler et lisser) le matériau de travail 4 ramolli sur la zone d'intervention du dispositif médical de type semelle S ou orthoplastie O.

Conformément à l'invention, le dispositif support 2 comprend au moins un module qui comprend lui-même une surface de réception adaptée à épouser au moins une partie de la première surface de dispositif (S1, O1), ou au moins une partie de la seconde surface de dispositif (S2, O2) ; et ce dispositif support 2 comprend encore des moyens de chauffage adaptés pour chauffer ladite surface de réception dudit au moins un module à une température au moins égale à ladite température de ramollissement du matériau constitutif dudit dispositif médical de type semelle S ou orthoplastie O.

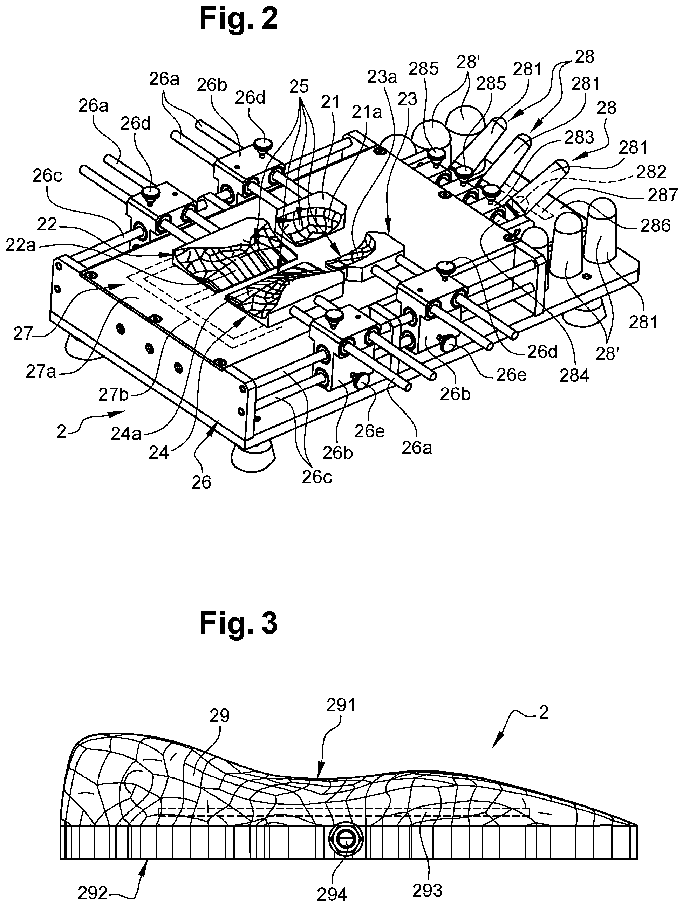

Tel qu'il apparait dans l'équipement 1 de la figure 1 et tel que représenté isolément sur la figure 2, le dispositif support 2 comprend différents modules, ici pour certains adaptés à la réception d'une semelle, et pour d'autres adaptés à la réception d'une orthoplastie.

Plus particulièrement, le dispositif support 2 comprend un premier groupe de modules 21, 22, 23 et 24 qui sont juxtaposés et qui comprennent chacun une partie de surface de réception 21a, 22a, 23a et 24a aptes ensemble à reconstituer une surface de réception 25 adaptée à épouser au moins une partie de la surface de dessous S2 d'un dispositif médical de type semelle S (de sorte à pouvoir intervenir sur la surface de dessus S1 de la semelle S).

Les différents modules 21, 22, 23 et 24 juxtaposés sont solidaires de moyens supports adaptés pour permettre d'ajuster leur positionnement en écartement les uns par rapport aux autres en vue de permettre l'adaptation de ladite surface de réception 25 à la pointure de la semelle S à recevoir.

Plus précisément, les modules 21, 22, 23 et 24 sont fixés sur un châssis porteur 26 par l'intermédiaire de tiges de guidage 26a montées à coulissement sur un chariot support 26b qui permet leur mobilité selon un premier axe ; et chaque chariot support 26b est lui-même monté coulissant sur une structure de glissière 26c selon un second axe qui est perpendiculaire audit premier axe.

Les tiges de guidage 26a peuvent être verrouillées sur leur chariot support 26b au moyen de vis de serrage 26d ; et les chariots support 26b peuvent être verrouillés sur leur structure de glissière 26c au moyen de vis de serrage 26e.

On comprend que les 4 modules juxtaposés 21, 22, 23 et 24 peuvent ainsi être écartés ou rapprochés les uns des autres, cela dans un même plan, pour optimiser la forme et les dimensions de la surface de réception 25 (composée de l'assemblage des quatre parties de surfaces de réception 21a, 22a, 23a et 24a), en fonction de la forme et des dimensions de la semelle S sur laquelle on souhaite intervenir.

On notera qu'une fois posée sur la surface de réception 25, la semelle S peut être maintenue en place par tout moyen approprié.

Pour cela on peut utiliser un ou plusieurs plots de maintien rapporté(s), par exemple en forme de bille (ou de sphère). De telles billes de maintien 2a sont illustrées sur la figure 1 et font partie de l'équipement 1. Elles sont de préférence réalisés en un matériau adapté pour sa coopération par aimantation avec les modules 21, 22, 23 et 24. On utilise par exemple des billes en néodyme, coopérant avec des modules 21, 22, 23 et 24 réalisés en acier (et plus particulièrement en acier recouvert d'une couche de protection contre la corrosion en zinc).

Le dispositif support 2 comprend des moyens de chauffage adaptés pour élever la température des modules 21, 22, 23 et 24, et donc celle de la semelle S réceptionnée par ces modules et sur laquelle on souhaite intervenir. Les moyens de chauffage correspondants sont adaptés/dimensionnés pour permettre de porter la zone de la semelle S sur laquelle on souhaite intervenir à la température de ramollissement du matériau (par exemple comprise entre 60 et 250°C, selon les matériaux).

Pour cela, les modules 21, 22, 23 et 24 sont réalisés en un matériau conducteur thermique (en acier par exemple, tel que mentionné ci-dessus) ; et ils peuvent être munis d'un système de résistance(s) électrique(s) relié à une alimentation électrique.

En variante, et tel que cela est prévu sur le dispositif support 2 des figures 1 et 2, les moyens de chauffage consistent en un plateau chauffant 27 en contact ou pratiquement en contact avec les modules 21, 22, 23 et 24.

Le plateau chauffant 27 est réalisé en matériau conducteur thermique et il comprend une face de dessus 27a, ainsi qu'une face de dessous opposée. Sa face de dessus 27a s'étend à proximité immédiate de la partie de dessous des modules 21, 22, 23 et 24, dans un plan parallèle au plan de déplacement de ces derniers guidés par les tiges de guidage 26a et les structures de glissières 26c. En outre, des moyens de chauffage en forme de résistances électriques 27b sont ménagés contre ou à proximité immédiate de sa face de dessous. La ou les résistances électrique 27b sont simplement schématisées sur la figure 2. Elles peuvent être reliées à une alimentation électrique constituée d'un câble électrique 2b et d'une armoire ou boite électrique 2c reliée au secteur.

A titre de variante de réalisation, seulement certains des modules juxtaposés 21, 22, 23 et 24 peuvent être montés sur des moyens support permettant leur ajustement en position, l'autre ou les autres modules étant prévus non mobiles.

Encore à titre de variante les différents modules 21, 22, 23 et 24 peuvent être dépourvus de moyens support et être simplement posés sur le plateau chauffant 27. Alors, ces modules 21, 22, 23 et 24 peuvent coopérer par aimantation avec le plateau 27 de réception. Par exemple on peut utiliser des modules 21, 22, 23 et 24 en néodyme, ou intégrant un noyau de néodyme pour coopérer avec le plateau 27 réalisé en acier.

Encore à titre de variante, un seul module peut être prévu (non mobile mais éventuellement amovible) pour constituer la surface de réception 25 adaptée pour recevoir au moins une partie de la surface de dessous S2 de la semelle S.

Les modules juxtaposés 21, 22, 23 et 24 peuvent aussi être adaptés pour reconstituer une surface de réception adaptée à épouser au moins une partie de la surface de dessus S1 d'un dispositif médical de type semelle S (de sorte à pouvoir intervenir sur la surface de dessous S2 de la semelle S).

Comme illustré sur les figures 1 et 2, le dispositif support 2 peut comprendre un second groupe de modules 28 adaptés à la réception d'une orthoplastie O.

Chaque module 28 comprend une surface de réception 281 adaptée à épouser au moins une partie de la surface interne O1 d'une orthoplastie O destinée à venir en contact avec la surface externe d'un doigt de pied (de sorte à pouvoir intervenir sur la surface externe O2 de l'orthoplastie O).

Les modules 28 d'orthoplastie du dispositif support 2 sont ici au nombre de 3, et ils se présentent chacun au moins approximativement sous la forme générale d'un organe cylindrique s'apparentant à un doigt de pied.

Chaque organe cylindrique 28 est monté par emboitement amovible sur une rotule support 282 solidaire d'un chariot support 283 ; et les différents chariots support 283 sont montés mobiles le long d'une même structure de glissière 284.

Chaque chariot support 283 comprend ses propres moyens de verrouillage amovible, en forme de vis de serrage 285, adaptés pour permettre de régler leur position sur la structure de glissière 284.

Le dispositif support 2 comprend des moyens de chauffage adaptés pour élever la température des modules 28, et donc celle de l'orthoplastie O réceptionnée et sur laquelle on souhaite intervenir. Les moyens de chauffage correspondants sont adaptés/dimensionnés pour permettre de porter la zone de l'orthoplastie O sur laquelle on souhaite intervenir à la température de ramollissement du matériau (par exemple de l'ordre de 60 à 250°C).

Pour cela, les modules 28 sont réalisés en un matériau conducteur thermique et ils peuvent être munis d'un système de résistance(s) électrique(s) reliée à une alimentation électrique.

En variante, et tel que cela est prévu sur le dispositif support 2, les moyens de chauffage consistent en un plateau chauffant 286 dont la face de dessous est équipée de résistances électriques 287 reliées à la boite électrique 2c via un câble électrique 2d (d'une manière identique ou similaire au plateau chauffant 27).

Le câble 2d peut aussi être relié à une boite électrique dédiée.

Les rotules 282 permettent la mobilité dans l'espace de chaque module 28 qui peut donc être placé en contact ou à proximité du plateau chauffant 286 pour assurer l'élévation en température de l'orthoplastie O associée.

Chaque module 28 permet la réception d'une orthoplastie mono-tubulaire ou d'un écarteur d'orteil. Et plusieurs modules 28 juxtaposés permettent la réception d'une orthoplastie complexe, comme par exemple une orthoplastie pluri-tubulaire ou attèle pour hallux valgus.

Le montage mobile des modules 28 le long de la structure de glissière 284 permet d'ajuster la distance entre chaque module de façon notamment à s'adapter aux cavités de l'orthoplastie tubulaire.

Les trois modules 28 juxtaposés peuvent avoir la même taille ou des tailles différentes. Leur montage amovible sur une rotule 282 permet leur remplacement aisé, en particulier pour en changer la taille en cas de besoin.

Comme on peut le voir sur les figures 1 et 2, le châssis porteur 26 du dispositif support 2 peut comporter une pluralité de modules orthoplastie 28' de remplacement, rangés sur des supports adaptés, en attente d'utilisation.

A titre de variante, le dispositif support 2 peut comporter uniquement un ou des modules adapté(s) à recevoir une semelle, ou uniquement un ou des modules adapté(s) à recevoir une orthoplastie.

D'autre part, chaque résistance électrique, ou chaque réseau de résistances électriques, dédié(e) aux modules « semelle » et aux modules « orthoplastie » peut comporter sa propre alimentation électrique (et donc, par exemple, être relié à sa propre boite électrique reliée au secteur).

Tel qu'illustré sur la figure 3, selon un autre mode de réalisation possible, ou selon un mode de réalisation complémentaire, le dispositif support 2 comprend un module 29 dont une surface de réception 291 est adaptée à épouser au moins une partie de la première surface de dispositif S1 d'un dispositif médical de type semelle S.

La surface de réception 291 de ce module 29 correspond à un dessous de pied ; et sa face inférieure 292 est plane pour pouvoir reposer de manière stable sur un support plan.

Ce module 29 comprend des moyens de chauffage adaptés pour élever sa température, et donc celle de la semelle S réceptionnée et sur laquelle on souhaite intervenir. Les moyens de chauffage correspondants sont adaptés/dimensionnés pour permettre de porter la zone de la semelle S sur laquelle on souhaite intervenir à la température de ramollissement du matériau (par exemple de l'ordre de 60 à 250°C).

Pour cela, le module 29 est réalisé en un matériau conducteur thermique (par exemple en acier), et il est muni d'un système de résistance(s) électrique(s) 293. Le système de résistance(s) électrique(s) 293 est relié à une alimentation électrique (par exemple via un câble électrique à la boite électrique 2c reliée au secteur, illustrée sur la figure 1, ou une boite électrique dédiée).

Ici, le système de résistance(s) électrique(s) 293 est intégré à l'intérieur du module 29 et on remarque l'ouverture 294 pour le passage de son câble d'alimentation.

En variante, les moyens de chauffage du module 29 peuvent consister en un plateau chauffant équipé de résistances électriques. On peut pour cela utiliser le plateau chauffant 27 du dispositif support des figures 1 et 2, ou un plateau chauffant dédié.

Le module 29 peut encore comprendre des moyens de fixation, pour sa fixation sur un support quelconque.

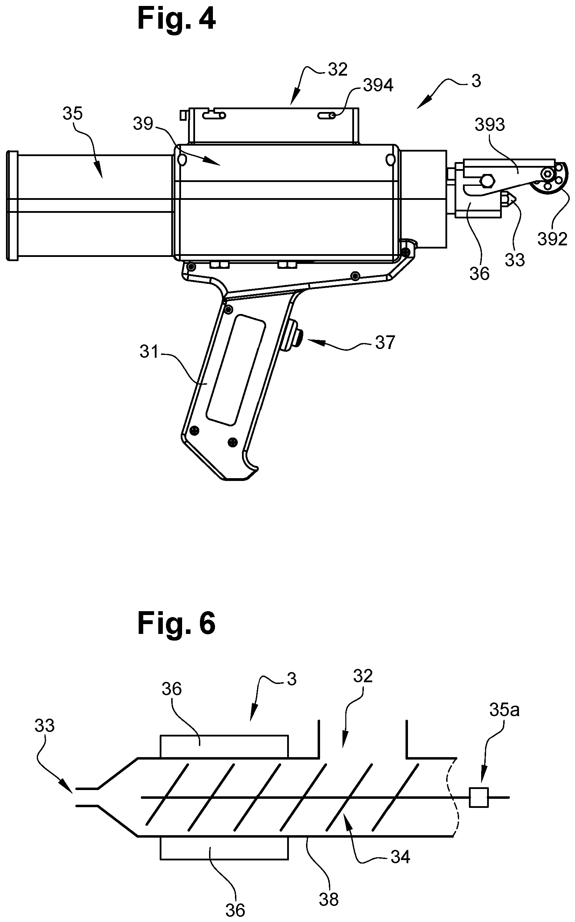

Les figures 4, 5 et 6 détaillent une forme de réalisation possible d'un dispositif de distribution 3 du matériau de travail 4.

Comme mentionné ci-dessus, le dispositif de distribution 3 est adapté pour recevoir le matériau de travail 4 sous forme de granulats solides (par exemple en poudre, paillettes, billes ou autre), pour chauffer ce matériau de travail 4 à une température permettant son ramollissement c'est-à-dire sa transformation en matériau pâteux ou semi-liquide, et pour distribuer ce matériau pâteux ou semi-liquide afin d'assurer sa dépose sur le dispositif médical à traiter.

Pour cela, le dispositif de distribution 3 comprend :
- un organe de préhension manuel en forme de poignée 31
- un logement de réception 32 pour la réception du matériau de travail 4,
- une buse de distribution 33 pour la distribution du matériau de travail 4 sous la forme d'un cordon de matière,
- des moyens de transfert en forme de vis motorisée 34, associée à une motorisation 35, pour transférer le matériau de travail 4 depuis le logement de réception 32 jusqu'à la buse de distribution 33,
- des moyens de chauffage 36, disposés entre le logement de réception 32 et la buse de distribution 33, adaptés pour porter le matériau de travail 4 à sa température de ramollissement,
- des moyens de commande 37 pour la commande de la motorisation 35 de la vis motorisée 34, en vue de la distribution, par la buse de distribution 33, du matériau de travail 4 ramolli.

La vis motorisée 34 est logée dans un fourreau de vis 38 qui aboutit à la buse de distribution 33. Cette vis motorisée 34 est entrainée en rotation par la motorisation 35 via un système d'accouplement 35a.

Les moyens de chauffage 36 comprennent une ou plusieurs résistances électriques intégrées dans un bloc en matériau conducteur thermique qui entoure le fourreau de vis 38.

L'ensemble constitué par le logement de réception 32, le fourreau de vis 38, la vis motorisée 34, la motorisation 35, les moyens de chauffage 36, et la buse de distribution 33, est logé dans un carter 39 qui se prolonge par la poignée 31.

Le carter 39 intègre des moyens de ventilation 391 adaptés pour réguler la température de la motorisation 35.

Les moyens de commande 37 consistent en une gâchette électrique ménagée au niveau de la poignée 31.

Le dispositif de distribution 3 est relié à une alimentation électrique pour assurer l'alimentation en électricité des moyens de commande 37 et des moyens de chauffage 36. Par exemple cette alimentation électrique est réalisée par un câble électrique 3a relié à la boite électrique 2c de l'équipement illustré sur la figure 1. Dans une variante de réalisation cette alimentation peut consister en une boite électrique dédiée.

Les moyens de chauffage 36 du dispositif de distribution 3 sont activés via un dispositif de régulation et un interrupteur se trouvant sur la boite électrique associée.

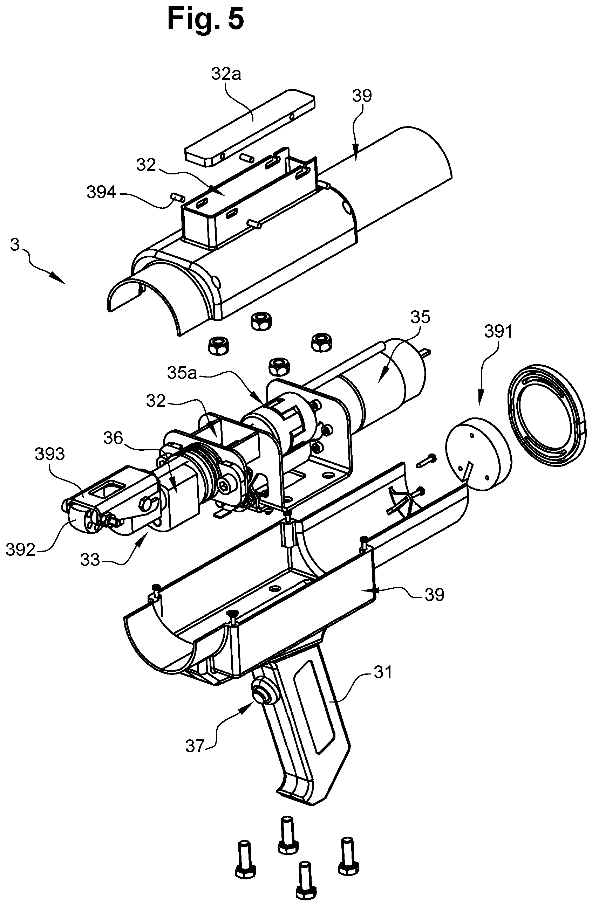

Sur les figures 4 et 5, on remarque la présence d'un organe de modelage 392 disposé dans le prolongement de la buse de distribution 33, qui est adapté pour permettre le modelage du matériau de travail 4 ramolli, une fois ce dernier déposé sur le dispositif médical.

L'organe de modelage 392 est fixé à l'extrémité d'un bras support 393 (de préférence amovible) monté en porte à faux au niveau de l'extrémité du dispositif de distribution 3. Il est disposé à quelques centimètres de la buse de distribution 33, légèrement décalé en retrait de l'axe de cette dernière.

L'organe de modelage 392 consiste ici en un patin fixe en forme générale de portion de cylindre. Dans une variante de réalisation il peut consister en un galet rotatif.

L'organe de modelage en forme de patin ou de galet 392 est de préférence réalisé en matériau bon conducteur thermique et insensible aux propriétés d'attraction des billes en néodyme 2a (on utilise pour cela un matériau tel que le zinc).

Le logement de réception 32 est de préférence équipé d'un couvercle 32a qui permet sa fermeture amovible. Le couvercle 32a est monté à pivotement autour d'un axe pivot 394 au niveau de l'une de ses extrémités, et il comporte des moyens de verrouillage en position fermée, par translation, au moyen d'ergots latéraux coopérant avec des gorges de réception.

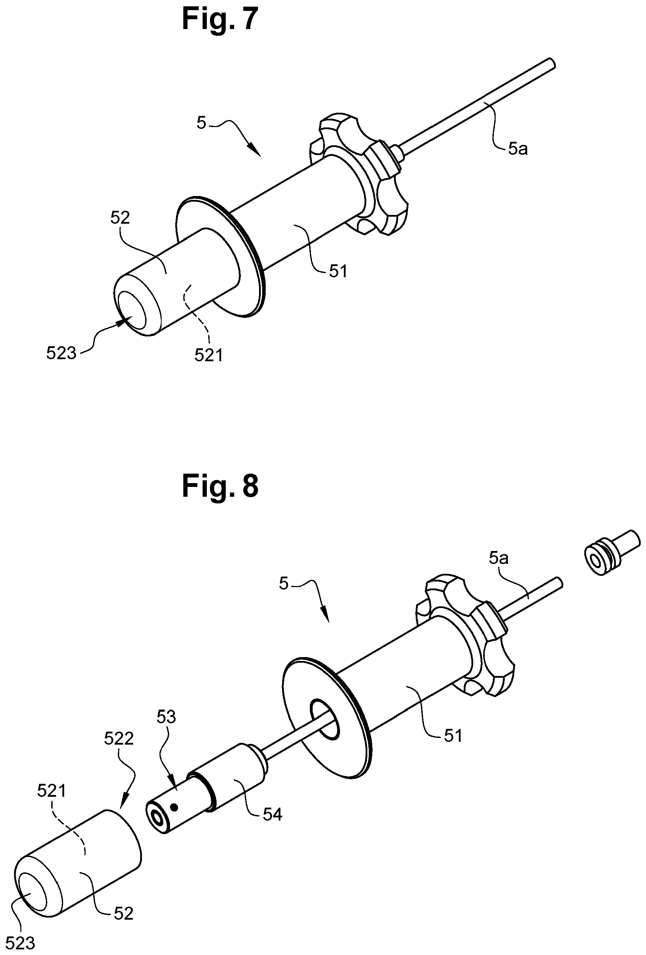

Les figures 7 et 8 détaillent une forme de réalisation possible d'un dispositif de façonnage 5 du matériau de travail 4 (après ramollissement de ce dernier).

Comme on peut le voir sur ces figures 7 et 8, le dispositif de façonnage 5 comprend ici : - un organe de préhension manuel 51 - un embout de façonnage 52 conformé pour permettre le façonnage du matériau de travail 4 ramolli, et - des moyens de chauffage 53 adaptés pour permettre de porter ledit embout de façonnage 52 à la température de ramollissement du matériau de travail 4 (à savoir de l'ordre de 180 à 250°C).

Le dispositif de façonnage 5 est relié à une alimentation électrique pour assurer l'alimentation en électricité des moyens de chauffage 53. Comme illustré sur la figure 1 cette alimentation électrique peut être constituée par un câble électrique 5a relié à la boite électrique 2c. Dans une variante de réalisation cette alimentation peut comprendre une boite électrique dédiée.

L'embout de façonnage 52 consiste en un corps creux dont la chambre interne 521 est munie d'une ouverture 522 à l'une de ses extrémités, et dans laquelle chambre interne 521 est logée au moins une résistance électrique chauffante constitutive des moyens de chauffage 53.

L'embout 52 est réalisé en matériau bon conducteur thermique, par exemple en zinc du fait de son insensibilité aux propriétés d'attraction des billes en néodyme 2a.

L'extrémité 523 de l'embout de façonnage 52, qui est opposée à celle munie de l'ouverture 522, est conformée de manière à optimiser le travail de façonnage à réaliser. Cette extrémité 523 peut être en forme de dôme, ou de plateau.

De préférence l'embout de façonnage 52 est prévu amovible sur le dispositif de façonnage 5 pour permettre son remplacement, par exemple en cas de détérioration, ou si on souhaite utiliser une autre forme d'embout en fonction de travail à réaliser.

Dans ce cas, l'embout de façonnage 52 comprend des moyens de fixation amovible (par exemple des moyens d'emboitement élastiques), soit sur l'organe de préhension manuel 51, soit sur un support 54 des moyens de chauffage 53.

L'équipement 1 tel que décrit ci-dessus permet de réparer ou de modifier un dispositif médical réalisé en matériau thermoformé comprenant une température de ramollissement, et qui comprend - une première surface de dispositif, anatomique, destinée à venir recouvrir une partie de corps d'un individu, et - une seconde surface de dispositif, opposée à ladite première surface de dispositif.

Le procédé en question peut comprendre :
- une étape de découpe d'une zone localisée sur ledit dispositif médical S, O, au moyen d'un outillage adapté (par exemple des ciseaux), pour obtenir un trou ou une cavité traversante délimitée par deux ouvertures de cavité (de chaque côté du dispositif médical) et par une bordure périphérique de cavité,
- une étape de positionnement du dispositif médical S, O sur le dispositif support 2 à disposition, pour fermer l'une des deux ouvertures de cavité,
- une étape de chauffage du dispositif médical S, O au moins au niveau du pourtour de la cavité traversante, le chauffage en question étant adapté pour obtenir le ramollissement du pourtour de la cavité traversante,
- une étape de comblement de la cavité traversante, au moins en partie par dépose du matériau de travail 4 ramolli par la chaleur, par l'ouverture de cavité non obturée, pour obtenir une structure de comblement de la cavité traversante (cette étape de dépose du matériau de travail 4 ramolli par la chaleur est de préférence réalisée au moyen du dispositif de distribution 3 tel que décrit ci-dessus),
- une étape de conformation de manière anatomique au moins de la face du matériau de travail de la structure de comblement destinée à venir en regard de la partie de pied concernée, au moyen d'un dispositif de façonnage (par exemple le dispositif de façonnage 5 tel que décrit ci-dessus).

L'étape de comblement de la cavité traversante peut être réalisée intégralement par du matériau de travail 4 ramolli par la chaleur.

Dans une variante de réalisation, cette étape de comblement de la cavité traversante est réalisée (a) en rapportant une pièce de matière solide, délimitée par une bordure périphérique, dans ladite cavité traversante en préservant un espace avec la bordure périphérique de ladite cavité traversante, et (b) en déposant le matériau de travail ramolli par la chaleur dans l'espace entre la bordure périphérique de la cavité traversante et la bordure périphérique de la pièce solide rapportée.

La pièce de matière solide rapportée consiste de préférence en une pièce d'un matériau identique à celui constitutif du dispositif médical S, O.

Le matériau de remplissage ramolli vient se solidariser par soudure avec la bordure périphérique de la cavité et le cas échéant avec la bordure périphérique de la pièce solide rapportée.

Encore selon une variante de réalisation, l'étape de comblement de la cavité traversante est réalisée en rapportant une pièce de matière solide, délimitée par une bordure périphérique, dans ladite cavité traversante sans préserver d'espace avec la bordure périphérique de ladite cavité traversante. La bordure périphérique de la cavité traversante et la bordure périphérique de la pièce solide rapportée sont alors en contact au niveau d'une ligne de contact ; et on peut ramollir les matières compatibles au niveau de cette ligne de contact (par exemple avec le dispositif de façonnage 5 ou avec l'organe de modelage 392 du dispositif de distribution 3), pour qu'elles fusionnent.

Comme précisé précédemment, l'invention peut également trouver application dans le domaine des dispositifs médicaux de type orthèses de correction optique, et en particulier des montures de lunettes, ou encore dans le domaine des prothèses, pour le remplacement d'un membre (ou partie de membre) ou d'une articulation.

On notera que le dispositif de distribution 3 décrit ci-dessus peut être utilisé isolément, indépendamment du dispositif support 2 et du dispositif de façonnage 5, par exemple pour d'autres applications, en vue de délivrer un cordon de matière ramollie (pâteuse/semi-liquide).

De la même manière, le dispositif de façonnage 5 décrit ci-dessus peut être utilisé isolément, indépendamment du dispositif support 2 et du dispositif de distribution 3, par exemple pour d'autres applications, en vue de façonner, étaler et/ou lisser un matériau de travail ramolli par la chaleur, déposé sur un support quelconque.

## Revendications

1. Dispositif support (2) pour la réception d'un dispositif médical de type orthèse ou prothèse, en vue de permettre une intervention de réparation ou de modification dudit dispositif médical de type orthèse ou prothèse, lequel dispositif médical est réalisé en matériau thermoformé comprenant une température de ramollissement, et lequel dispositif médical comprend - une première surface de dispositif, anatomique, destinée à venir recouvrir une partie de corps d'un individu, et - une seconde surface de dispositif, opposée à ladite première surface de dispositif,
lequel dispositif support (2) comprend au moins un module (21, 22, 23, 24) comprenant une surface de réception (25) adaptée à épouser au moins une partie de ladite première surface de dispositif, ou au moins une partie de ladite seconde surface de dispositif, lequel dispositif support (2) comprend encore des moyens de chauffage (27b, 287, 293) adaptés pour chauffer ladite surface de réception (25) dudit au moins un module (21, 22, 23, 24) à une température au moins égale à ladite température de ramollissement du matériau constitutif dudit dispositif médical de type orthèse ou prothèse,
**caractérisé en ce que** ledit dispositif support (2) comprend une pluralité de modules (21, 22, 23, 24) juxtaposés, lesquels modules (21, 22, 23, 24) comprennent chacun une partie de surface de réception (21a, 22a, 23a et 24a) de ladite surface de réception (25) adaptée à épouser au moins une partie de ladite première surface de dispositif d'une orthèse type semelle, ou au moins une partie de ladite seconde surface de dispositif d'une orthèse de type semelle,
lesquels modules (21, 22, 23, 24) sont aptes ensemble à reconstituer ladite surface de réception (25), au moins certains desdits modules (21, 22, 23, 24) étant solidaires de moyens supports (26a, 26b, 26c) adaptés pour permettre d'ajuster leur positionnement en écartement les uns par rapport aux autres en vue de permettre l'adaptation de ladite surface de réception (25) à la pointure du dispositif médical de type semelle.

2. Dispositif support (2) selon la revendication 1, **caractérisé en ce qu'**il comprend en outre au moins un module (28) qui se présente au moins approximativement sous la forme générale d'un organe cylindrique, s'apparentant à un doigt de pied, et dont la surface externe constitue une surface de réception (281) adaptée à épouser au moins une partie de ladite seconde surface de dispositif d'un dispositif médical de type orthoplastie, ledit ou lesdits organes cylindriques (28) étant montés par emboitement amovible sur une rotule (282) support permettant leur mobilité dans l'espace.

3. Dispositif support (2) selon la revendication 2, **caractérisé en ce qu'**il comprend une pluralité d'organes cylindriques (28) portés chacun par un chariot support (283), lesquels chariots support (283) sont montés mobiles sur une structure de glissière (284) et comprennent chacun leurs propres moyens de verrouillage amovible (285), adaptés pour permettre de régler leur position sur ladite structure de glissière (284).

4. Dispositif support (2) selon la revendication 1, **caractérisé en ce qu'**il comprend un module (29) dont la surface de réception (291) adaptée à épouser au moins une partie d'une première surface de dispositif d'une orthèse type semelle, ou au moins une partie d'une seconde surface de dispositif d'une orthèse de type semelle.

5. Dispositif support (2) selon la revendication 1, **caractérisé en ce que** au moins certains desdits modules (21, 22, 23, 24) sont fixés sur des tiges de guidage (26a) montées à coulissement sur un chariot support (26b) pour permettre leur mobilité selon un premier axe, lequel chariot support (26b) est lui-même monté coulissant sur une structure de glissière (26c) selon un second axe perpendiculaire audit premier axe.

6. Dispositif support (2) selon l'une quelconque des revendications 1 à 5, **caractérisé en ce que** ledit module (21, 22, 23, 24 ; 28 ; 29) ou l'un au moins desdits modules (21, 22, 23, 24 ; 28 ; 29) est réalisé en matériau conducteur thermique et comprend lesdits moyens de chauffage en forme d'au moins une résistance électrique (293).

7. Dispositif support (2) selon l'une quelconque des revendications 1 à 6, **caractérisé en ce qu'**il comprend au moins un plateau chauffant (27, 286) en matériau conducteur thermique muni d'une face de dessous et d'une face de dessus, lesdits moyens de chauffage en forme d'au moins une résistance électrique (27b, 287) étant ménagés à proximité de ladite face de dessous, et **en ce que** ledit module (21, 22, 23, 24 ; 28 ; 29) ou l'un au moins desdits modules (21, 22, 23, 24 ; 28 ; 29) est disposé ou apte à être disposé en contact avec ladite face de dessus dudit plateau chauffant (27, 286) ou juste à proximité de ce dernier, pour permettre son chauffage par conduction.

8. Equipement (1) pour réparer ou modifier un dispositif médical de type orthèse ou prothèse, lequel dispositif médical est réalisé en matériau thermoformé comprenant une température de ramollissement, et lequel dispositif médical comprend - une première surface de dispositif, anatomique, destinée à venir recouvrir une partie de corps d'un individu, et - une seconde surface de dispositif, opposée à ladite première surface de dispositif, **caractérisé en ce qu'**il comprend :
- un dispositif support (2) selon l'une quelconque des revendications 1 à 7,
- un matériau de travail (4) thermoformable, comprenant une température de ramollissement, de consistance solide à la température ambiante et apte par chauffage à prendre une consistance semi liquide ou pâteuse à ladite température de ramollissement, et
- un dispositif de façonnage (5) adapté pour mettre en forme ledit matériau de travail (4) sur ledit dispositif médical.

9. Equipement (1) selon la revendication 8, **caractérisé en ce que** ledit matériau de travail (4) comprend une température de ramollissement identique ou proche de la température de ramollissement du matériau constitutif du dispositif médical).

10. Equipement (1) selon l'une quelconque des revendications 8 ou 9, **caractérisé en ce que** ledit dispositif de façonnage (5) comprend :
- un organe de préhension manuel (51),
- un embout de façonnage (52) conformé pour permettre le façonnage du matériau de travail (4) et
- des moyens de chauffage (53) adaptés pour permettre de porter ledit embout de façonnage (52) à ladite température de ramollissement du matériau de travail (4).

11. Equipement (1) selon l'une quelconque des revendications 8 à 10, **caractérisé en ce qu'**il comprend un dispositif de distribution (3) dudit matériau de travail (4), lequel dispositif de distribution (3) comprend :
- un organe de préhension manuel (31),
- un logement de réception (32) pour la réception dudit matériau de travail (4) sous forme solide,
- une buse de distribution (33) pour la distribution dudit matériau de travail (4),
- des moyens de transfert en forme de vis motorisée (34), associée à une motorisation (35), pour transférer ledit matériau de travail (4) depuis ledit logement de réception (32) jusqu'à ladite buse de distribution (33),
- des moyens de chauffage (36), disposés entre ledit logement de réception (32) et ladite buse de distribution (33), adaptés pour porter ledit matériau de travail (4) à sa température de ramollissement,
- des moyens de commande (37) de ladite motorisation (35) de la vis motorisée (34) pour la distribution par ladite buse de distribution (33) du matériau de travail (4) ramolli.

12. Equipement (1) selon la revendication 11, **caractérisé en ce que** ledit dispositif de distribution (3) comprend un organe de modelage (392) en forme de patin fixe ou de galet rotatif, disposé dans le prolongement de ladite buse de distribution (33).

13. Procédé pour réparer ou modifier un dispositif médical de type orthèse ou prothèse, au moyen d'un équipement (1) selon l'une quelconque des revendications 8 à 12, lequel dispositif médical est réalisé en matériau thermoformé comprenant une température de ramollissement, et lequel dispositif médical comprend - une première surface de dispositif, anatomique, destinée à venir recouvrir une partie de corps d'un individu, et - une seconde surface de dispositif, opposée à ladite première surface de dispositif,
lequel procédé comprend :
- une étape de découpe d'une zone localisée sur ledit dispositif médical, au moyen d'un outillage adapté, pour obtenir une cavité traversante délimitée par deux ouvertures de cavité et par une bordure périphérique de cavité,
- une étape de positionnement dudit dispositif médical sur ledit dispositif support (2) pour fermer l'une desdites deux ouvertures de cavité,
- une étape de chauffage dudit dispositif médical au moins au niveau du pourtour de ladite cavité traversante, adaptée pour obtenir le ramollissement dudit pourtour de ladite cavité traversante,
- une étape de comblement de ladite cavité traversante, au moins en partie par dépose dudit matériau de travail (4) ramolli par la chaleur, par l'ouverture de cavité non obturée, pour obtenir une structure de comblement de ladite cavité traversante,
- une étape de conformation de manière anatomique au moins de la face du matériau de travail (4) de la structure de comblement destinée à venir en regard de la partie de corps concernée, au moyen dudit dispositif de façonnage (5).

14. Procédé selon la revendication 13, **caractérisée en ce que** ladite étape de comblement de ladite cavité traversante est réalisée (a) en rapportant une pièce de matière solide, délimitée par une bordure périphérique, dans ladite cavité traversante en préservant un espace avec la bordure périphérique de ladite cavité traversante, et (b) en déposant ledit matériau de travail (4) ramolli par la chaleur dans l'espace entre la bordure périphérique de la cavité traversante et la bordure périphérique de la pièce solide rapportée.

## Patentansprüche

1. Haltevorrichtung (2) zum Aufnehmen einer medizinischen Vorrichtung vom Typ Orthese oder Prothese, um einen Eingriff zum Reparieren oder Abändern der medizinischen Vorrichtung vom Typ Orthese oder Prothese zu ermöglichen, wobei die medizinische Vorrichtung aus einem thermogeformten Material mit einer Erweichungstemperatur hergestellt ist und wobei die medizinische Vorrichtung - eine erste, anatomische Vorrichtungsoberfläche, die dazu bestimmt ist, einen Teil des Körpers einer Person zu bedecken, und - eine zweite, von der ersten Vorrichtungsoberfläche abgewandte Vorrichtungsoberfläche aufweist,
wobei die Haltevorrichtung (2) mindestens ein Modul (21, 22, 23, 24), das eine zum Anliegen an mindestens einem Teil der ersten Vorrichtungsoberfläche oder an mindestens einem Teil der zweiten Vorrichtungsoberfläche ausgelegte Aufnahmeoberfläche (25) aufweist, wobei die Haltevorrichtung (2) außerdem Heizmittel (27b, 287, 293) aufweist, die zum Heizen der Aufnahmeoberfläche (25) des mindestens einen Moduls (21, 22, 23, 24) auf eine Temperatur, die mindestens gleich der Erweichungstemperatur des Materials ist, aus dem die medizinische Vorrichtung vom Typ Orthese oder Prothese besteht, ausgelegt ist,
**dadurch gekennzeichnet, daß** die Haltevorrichtung (2) eine Vielzahl nebeneinander angeordneter Module (21, 22, 23, 24) aufweist, wobei die Module (21, 22, 23, 24) jeweils einen Aufnahmeoberflächenteil (21a, 22a, 23a, 24a) der Aufnahmeoberfläche (25) aufweisen, der dazu ausgelegt ist, an mindestens einem Teil der ersten Vorrichtungsoberfläche einer Orthese vom Typ einer Sohle oder an mindestens einem Teil der zweiten Vorrichtungsoberfläche einer Orthese vom Typ einer Sohle anzuliegen,
wobei die Module (21, 22, 23, 24) geeignet sind, gemeinsam die Aufnahmeoberfläche (25) wiederherzustellen, wobei mindestens einige der Module (21, 22, 23, 24) an Haltemitteln (26a, 26b, 26c) befestigt sind, die dazu ausgelegt sind, deren gegenseitige abstandsmäßige Positionierung zu ermöglichen, um die Anpassung der Aufnahmeoberfläche (25) an die Schuhgröße der medizinischen Vorrichtung vom Typ Sohle zu ermöglichen.

2. Haltevorrichtung (2) gemäß Anspruch 1, **dadurch gekennzeichnet, daß** sie außerdem mindestens ein Modul (28) aufweist, das mindestens näherungsweise in der allgemeinen Form eines zylindrischen Organs vorliegt, das einer Zehe ähnelt und dessen äußere Oberfläche eine Aufnahmeoberfläche (21) bildet, die dazu ausgelegt ist, an mindestens einem Teil der zweiten Vorrichtungsoberfläche einer medizinischen Vorrichtung vom Typ einer Orthoplastik anzuliegen, wobei das oder die zylindrische(n) Organ(e) (28) abnehmbar auf ein Tragegelenk (282) aufgesteckt ist bzw. sind, das dessen (deren) räumliche Beweglichkeit ermöglicht.

3. Haltevorrichtung (2) gemäß Anspruch 2, **dadurch gekennzeichnet, daß** sie eine Vielzahl zylindrischer Organe (28) aufweist, die jeweils von einem Trageschlitten (283) gehalten sind, wobei die Trageschlitten (283) auf einer Gleitschienenstruktur (284) beweglich angebracht sind und jeweils deren eigene abnehmbare Verriegelungsmittel (285) aufweisen, die dazu ausgelegt sind, deren Position auf der Gleitschienenstruktur (284) einzustellen.

4. Haltevorrichtung (2) gemäß Anspruch 1, **dadurch gekennzeichnet, daß** sie ein Modul (29) aufweist, deren Aufnahmeoberfläche (291) dazu ausgelegt ist, an mindestens einem Teil einer ersten Vorrichtungsoberfläche einer Orthese vom Typ einer Sohle oder mindestens an einem Teil einer zweiten Vorrichtungsoberfläche einer Orthese vom Typ einer Sohle anzuliegen.

5. Haltevorrichtung (2) gemäß Anspruch 1, **dadurch gekennzeichnet, daß** mindestens einige der Module (21, 22, 23, 24) auf Führungsstäben (26a) befestigt sind, die gleitend auf einem Trageschlitten (26b) angebracht sind, um deren Beweglichkeit entlang einer ersten Achse zu ermöglichen, wobei der Trageschlitten (26b) selbst auf einer Gleitstruktur (26c) entlang einer zur ersten Achse senkrechten zweiten Achse beweglich angebracht ist.

6. Haltevorrichtung (2) gemäß einem der Ansprüche 1 bis 5, **dadurch gekennzeichnet, daß** das Modul (21, 22, 23, 24; 28; 29) oder mindestens eins der Module (21, 22, 23, 24; 28; 29) aus einem wärmeleitenden Material hergestellt ist und die besagten Heizmittel in Form mindestens eines elektrischen Widerstands (293) aufweist.

7. Haltevorrichtung (2) gemäß einem der Ansprüche 1 bis 6, **dadurch gekennzeichnet, daß** sie mindestens eine Heizplatte (27, 286) aus einem wärmeleitenden Material mit einer Unterseite und einer Oberseite aufweist, wobei die Heizmittel in Form mindestens eines elektrischen Widerstands (27b, 287) nahe der Unterseite angeordnet sind, und daß das Modul (21, 22, 23, 24; 28; 29) oder mindestens eins der Module (21, 22, 23, 24; 28; 29) in Kontakt mit der Oberseite der Heizplatte (27, 286) oder nur in deren Nähe angeordnet ist oder angeordnet werden kann, um dessen Aufheizen durch Leitung zu ermöglichen.

8. Ausrüstung (1) zum Reparieren oder Abändern einer medizinischen Vorrichtung vom Typ Orthese oder Prothese, wobei die medizinische Vorrichtung aus einem thermogeformten Material mit einer Erweichungstemperatur hergestellt ist und wobei die medizinische Vorrichtung - eine erste, anatomische Vorrichtungsoberfläche, die dazu bestimmt ist, einen Teil des Körpers einer Person zu bedecken, und - eine zweite, von der ersten Vorrichtungsoberfläche abgewandte Vorrichtungsoberfläche aufweist, **dadurch gekennzeichnet, daß** sie
- eine Haltevorrichtung (2) gemäß einem der Ansprüche 1 bis 7,
- ein thermoverformbares Material (4) mit einer Erweichungstemperatur, bei Raumtemperatur fester Konsistenz und einer bei der Erweichungstemperatur halbflüssigen oder pastenartigen Konsistenz und
- eine formgebende Vorrichtung (5), die geeignet ist, das Arbeitsmaterial (4) auf der medizinischen Vorrichtung zu formen,
aufweist.

9. Ausrüstung (1) gemäß Anspruch 8, **dadurch gekennzeichnet, daß** das Arbeitsmaterial (4) eine Erweichungstemperatur aufweist, die gleich oder nahe der Erweichungstemperatur des Materials ist, aus dem die medizinische Vorrichtung besteht.

10. Ausrüstung (1) gemäß einem der Ansprüche 8 oder 9, **dadurch gekennzeichnet, daß** die formgebende Vorrichtung (5)
- ein Organ (51) zum Erfassen von Hand,
- einen formgebenden Vorsatz (52), der dazu ausgelegt ist, die Formgebung des Arbeitsmaterials (4) zu ermöglichen, und
- Heizmittel (53), die geeignet sind, den formgebenden Vorsatz (52) auf die Erweichungstemperatur des Arbeitsmaterials (4) zu bringen,
aufweist.

11. Ausrüstung (1) gemäß einem der Ansprüche 8 bis 10, **dadurch gekennzeichnet, daß** sie eine Vorrichtung (3) zum Verteilen des Arbeitsmaterials (4) aufweist, wobei die Verteilvorrichtung (3)
- ein Organ (31) zum Erfassen von Hand,
- einen Aufnahmeplatz (32) für die Aufnahme des Arbeitsmaterials (4) in fester Form,
- eine Verteilerdüse (33) für die Verteilung des Arbeitsmaterials (4),
- Transportmittel in Form einer Motorisierungsmitteln (35) zugeordneten motorisierten Schraube (34), um das Arbeitsmaterial (4) vom Aufnahmeplatz (32) zur Verteilerdüse (33) zu bringen,
- zwischen dem Aufnahmeplatz (32) und der Verteilerdüse (33) angeordnete Heizmittel (36), die geeignet sind, das Arbeitsmaterial (4) auf seine Erweichungstemperatur zu bringen,
- Mittel (37) zum Steuern der Motorisierung (35) der motorisierten Schraube (34) für das Verteilen des erweichten Arbeitsmaterials (4) durch die Verteilerdüse (33) aufweist.

12. Ausrüstung (1) gemäß Anspruch 11, **dadurch gekennzeichnet, daß** die Verteilvorrichtung (3) ein Modellierorgan (392) in Form eines festen Gleiters oder einer drehenden Rolle aufweist, der beziehungsweise die in Verlängerung der Verteilerdüse (33) angeordnet ist.

13. Verfahren zum Reparieren oder Abändern einer medizinischen Vorrichtung vom Typ Orthese oder Prothese mittels einer Ausrüstung (1) gemäß einem der Ansprüche 8 bis 12, wobei die medizinische Vorrichtung aus einem thermogeformten Material mit einer Erweichungstemperatur hergestellt ist und wobei die medizinische Vorrichtung - eine erste, anatomische Vorrichtungsoberfläche, die dazu bestimmt ist, einen Teil des Körpers einer Person zu bedecken, und - eine zweite, von der ersten Vorrichtungsoberfläche abgewandte Vorrichtungsoberfläche aufweist, wobei das Verfahren aufweist:
- einen Schritt des Abschneidens eines auf der medizinischen Vorrichtung festgelegten Bereichs mittels eines geeigneten Werkzeugs, um einen durch zwei Hohlraumöffnungen und einen umlaufenden Hohlraumrand begrenzten durchgehenden Hohlraum zu erhalten,
- einen Schritt des Positionierens der medizinischen Vorrichtung auf der Haltevorrichtung (2), um eine der beiden Hohlraumöffnungen zu schließen,
- einen Schritt des Aufheizens der medizinischen Vorrichtung mindestens im Bereich des Umfangs des durchgehenden Hohlraums, der geeignet ist, das Erweichen des Umfangs des durchgehenden Hohlraums zu erreichen,
- einen Schritt des mindestens teilweisen Auffüllens des durchgehenden Hohlraums durch Einbringen des durch die Hitze erweichten Arbeitsmaterials (4) durch die nicht verschlossene Öffnung, um eine Auffüllstruktur des durchgehenden Hohlraums zu erhalten,
- einen Schritt des anatomischen Formens mittels der formgebenden Vorrichtung (5) mindestens der Seite des Arbeitsmaterials (4), die dazu bestimmt ist, gegenüber dem betreffenden Körperteil zu sein.

14. Verfahren gemäß Anspruch 13, **dadurch gekennzeichnet, daß** der Schritt des Auffüllens des durchgehenden Hohlraums ausgeführt wird, indem (a) ein durch einen umlaufenden Rand begrenztes Teil aus festem Material in den durchgehenden Hohlraum eingeführt wird und dabei ein Abstand zum umlaufenden Rand des durchgehenden Hohlraums bewahrt wird, und (b) das durch Hitze erweichte Arbeitsmaterial (4) in den Raum zwischen dem umlaufenden Rand des durchgehenden Hohlraums und dem umlaufenden Rand des eingefügten festen Teils eingebracht wird.

## Claims

1. A support device (2) for receiving a medical device such as an orthosis or a prosthesis, with a view to allowing a repair or modification procedure of said medical device such as an orthosis or a prosthesis, the medical device being made of thermoformed material comprising a softening temperature, and the medical device comprising - a first, anatomical, device surface, intended to cover a part of an individual's body, and - a second device surface, opposite said first device surface,
the support device (2) comprising at least one module (21, 22, 23, 24) comprising a receiving surface (25) adapted to conform to at least a part of said first device surface, or at least a part of said second device surface, the support device (2) further comprising heating means (27b, 287, 293) adapted to heat said receiving surface (25) of said at least one module (21, 22, 23, 24) to a temperature at least equal to said softening temperature of the constituent material of said medical device such as an orthosis or a prosthesis,
**characterized in that** said support device (2) comprises a plurality of juxtaposed modules (21, 22, 23, 24), the modules (21, 22, 23, 24) each comprising a receiving surface part (21a, 22a, 23a and 24a) of said receiving surface (25) adapted to conform to at least a part of said first device surface of an orthosis such as a sole, or at least a part of said second device surface of an orthosis such as a sole,
the modules (21, 22, 23, 24) being capable of reconstituting said receiving surface (25) together, at least some of said modules (21, 22, 23, 24) being integral with support means (26a, 26b, 26c) adapted to make it possible to adjust their spacing positioning relative to each another with a view to making it possible to adapt said receiving surface (25) to the size of the medical device such as a sole.

2. The support device (2) of claim 1, **characterized in that** it further comprises at least one module (28) which is at least approximately in the general form of a cylindrical member, resembling a toe, and the outer surface of which forms a receiving surface (281) adapted to conform to at least a part of said second device surface of a medical device such as an orthoplasty, said cylindrical member(s) (28) being mounted by removable interlocking on a support ball joint (282) allowing their spatial mobility.

3. The support device (2) of claim 2, **characterized in that** it comprises a plurality of cylindrical members (28) each borne by a support carriage (283), the support carriages (283) being movably mounted on a slide structure (284) and each comprising their own removable locking means (285), adapted to make it possible to adjust their position on said slide structure (284).

4. The support device (2) of claim 1, **characterized in that** it comprises a module (29) of which the receiving surface (291) is adapted to conform to at least a part of a first device surface of an orthosis such as a sole, or at least a part of a second device surface of an orthosis such as a sole.

5. The support device (2) of claim 1, **characterized in that** at least some of said modules (21, 22, 23, 24) are fastened to guide rods (26a) slidably mounted on a support carriage (26b) to allow their mobility along a first axis, the support carriage (26b) being itself slidably mounted on a slide structure (26c) along a second axis perpendicular to said first axis.

6. The support device (2) according to any of claims 1 to 5, **characterized in that** said module (21, 22, 23, 24; 28; 29) or at least one of said modules (21, 22, 23, 24; 28; 29) is made of thermally conductive material and comprises said heating means in the form of at least one electrical heating resistor (293).

7. The support device (2) according to any of claims 1 to 6, **characterized in that** it comprises at least one heating plate (27, 286) made of thermally conductive material provided with a bottom face and a top face, said heating means in the form of at least one electrical resistor (27b, 287) being arranged in proximity to said bottom face, and **in that** said module (21, 22, 23, 24; 28; 29) or at least one of said modules (21, 22, 23, 24; 28; 29) is disposed or capable of being disposed in contact with said top face of said heating plate (27, 286) or just in proximity to the latter, to allow its heating by conduction.

8. Equipment (1) for repairing or modifying a medical device such as an orthosis or a prosthesis, the medical device being made of thermoformed material comprising a softening temperature, and the medical device comprising - a first, anatomical, device surface, intended to cover a part of an individual's body, and - a second device surface, opposite said first device surface, **characterized in that** it comprises:
- a support device (2) according to any of claims 1 to 7,
- a thermoformable working material (4), comprising a softening temperature, of solid consistency at ambient temperature and capable by heating of adopting a semi-liquid or pasty consistency at said softening temperature, and
- a shaping device (5) adapted to shape said working material (4) on said medical device.

9. The item of equipment (1) of claim 8, **characterized in that** said working material (4) comprises a softening temperature identical or similar to the softening temperature of the constituent material of the medical device.

10. The item of equipment (1) according to any of claims 8 or 9, **characterized in that** said shaping device (5) comprises:
- a manual gripping member (51),
- a shaping tip (52) shaped to allow shaping of the working material (4) and
- heating means (53) adapted to make it possible to heat said shaping tip (52) to said softening temperature of the working material (4).

11. The item of equipment (1) according to any of claims 8 to 10, **characterized in that** it comprises a dispensing device (3) of said working material (4), the dispensing device (3) comprising:
- a manual gripping member (31),
- a receiving housing (32) for receiving said working material (4) in solid form,
- a dispensing nozzle (33) for dispensing said working material (4),
- motorized screw-shaped transfer means (34), associated with a motorization (35), for transferring said working material (4) from said receiving housing (32) to said dispensing nozzle (33),
- heating means (36), disposed between said receiving housing (32) and said dispensing nozzle (33), adapted to heat said working material (4) to its softening temperature,
- control means (37) of said motorization (35) of the motorized screw (34) for dispensing, via said dispensing nozzle (33), the softened working material (4).

12. The item of equipment (1) of claim 11, **characterized in that** said dispensing device (3) comprises a modeling member (392) in the form of a fixed pad or a rotary roller, disposed extending from said dispensing nozzle (33).

13. A method for repairing or modifying a medical device such as an orthosis or a prosthesis, by means of an item of equipment (1) according to any of claims 8 to 12, the medical device being made of thermoformed material comprising a softening temperature, and the medical device comprising - a first, anatomical, device surface, intended to cover a part of an individual's body, and - a second device surface, opposite said first device surface,
the method comprising:
- a step of cutting out a zone located on said medical device, by means of a suitable tool, to obtain a through cavity delimited by two cavity openings and by a peripheral cavity edge,
- a step of positioning said medical device on said support device (2) to close one of said two cavity openings,
- a step of heating said medical device at least at the perimeter of said through cavity, adapted to obtain softening of said perimeter of said through cavity,
- a step of filling said through cavity, at least in part by depositing said heat-softened working material (4), through the unsealed cavity opening, to obtain a filling structure of said through cavity,
- a step of anatomically shaping at least the face of the working material (4) of the filling structure intended to face the part of the body concerned, by means of said shaping device (5).

14. The method of claim 13, **characterized in that** said step of filling said through cavity is carried out (a) by mounting a piece of solid material, delimited by a peripheral edge, into said through cavity while keeping a gap with the peripheral edge of said through cavity, and (b) by depositing said heat-softened working material (4) in the gap between the peripheral edge of the through cavity and the peripheral edge of the mounted solid piece.
